(19) Europäisches Patentamt
European Patent Office
Office européen des brevets

(11) Numéro de publication: 0 281 471
B1

# (12) FASCICULE DE BREVET EUROPEEN

(45) Date de publication du fascicule du brevet: **02.05.90**

(51) Int. Cl.⁴: **C07D 307/83**, C07D 405/12, C07D 407/12, A61K 31/365

(21) Numéro de dépôt: **88400442.5**

(22) Date de dépôt: **26.02.88**

(54) **Nouveaux acétamides dérivés de la dihydro-2,3 phényl-3 benzofurannone-2, leurs procédés de préparation et les compositions pharmaceutiques qui les contiennent.**

(30) Priorité: **27.02.87 FR 8702631**

(43) Date de publication de la demande:
**07.09.88 Bulletin 88/36**

(45) Mention de la délivrance du brevet:
**02.05.90 Bulletin 90/18**

(84) Etats contractants désignés:
**AT BE CH DE FR GB IT LI LU NL SE**

(56) Documents cités:
**EP-A- 0 176 309**

**CHEMICAL ABSTRACTS, vol. 93, no. 5, 4 août 1980, page 108, résumé no. 37433q, Columbus, Ohio, US; V. MacMILLAN: "Effects of gamma-hydroxybutyrate and gamma-butyrolactone on cerebral energy metabolism during exposure and recovery from hypoxemia-oligemia", & STROKE 1980, 11(3), 271-7 000**

(73) Titulaire: **ADIR ET COMPAGNIE, 22, rue Garnier, F-92201 Neuilly sur Seine(FR)**

(72) Inventeur: **Lavielle, Gilbert, 1 Avenue Lilly, F-78170 La Celle Saint-Cloud(FR)**
Inventeur: **Lepagnol, Jean, 5 Rue M. De Vlamick, F-78400 Chatou(FR)**

**EP 0 281 471 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de la dihydro-2,3 phényl-3 benzofurannone-2, leurs procédés de préparation et les compositions pharmaceutiques qui les contiennent.

D'une façon surprenante, la littérature mentionne peu de dérivés de la dihydro-2,3 benzofurannone-2 pharmacologiquement actifs. Quelques esters de l'acide (dihydro-2,3 oxo-2 phényl-3 benzofurannyl-5)-2 acétique ayant des propriétés antiinflammatoires ont été décrits par Shridhar et col. dans Indian. J. Chem. (1980), vol 19B (10), p 891-893.

La demanderesse a maintenant découvert que certains acétamides dérivés de la dihydro-2,3 phényl-3 benzofurannone-2 possèdent des propriétés pharmacologiques très intéressantes.

En effet, les composés de la présente invention exercent des effets antihypoxiques et nootropes sans intervention des effets vasculaires. Ils s'opposent nettement à la mort cérébrale et à la défaillance énergétique tissulaire lors d'insuffisance d'apport en oxygène et trouvent leur application dans la correction des désordres liés à l'hypoxémie et à l'insuffisance énergétique, par exemple lors du vieillissement cérébral.

La présente invention a plus particulièrement pour objet les composés de formule générale I,

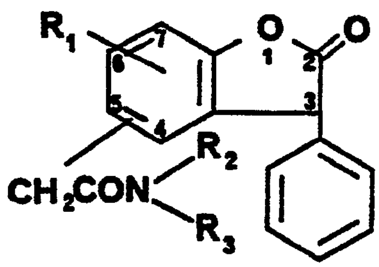

(I)

dans laquelle:

ou $R_1$ est en position 7, et le groupement acétamido est en position 5, ou $R_1$ est en position 5, le groupement acétamido es en position 7,

$R_1$ représente un atome d'hydrogène ou d'halogène,

$R_2$ et $R_3$ identiques ou différents représentent chacun un atome d'hydrogène ou un radical alkyle linéaire ou ramifié contenant 1 à 4 atomes de carbone, un radical benzyl éventuellement substitué par un atome d'halogène par un radical alcoxy contenant 1 à 4 atomes de carbone ou par un radical alkyle de 1 à 4 atomes de carbone, un radical méthylènedioxy-3,4 benzyle, ou forment ensemble avec l'azote auquel its sont attachés un radical morpholinyle-4, ou un radical pipérazinyle-1 (éventuellement substitué en 4 par un radical alkyle de 1 à 4 atomes de carbone, un radical benzyle éventuellement substitué par un radical alkyle de 1 à 4 atomes de carbone, un radical méthylènedioxy-3,4 benzyle, ou un radical pyridyle-2 éventuellement substitué par un radical alkyl de 1 à 4 atomes de carbone ou un radical trifluorométhyle),

et leurs sels d'addition à un acide minéral ou organique, pharmaceutiquement acceptable.

La présente invention a également pour objet le procédé de préparation des composés de formule générale I, caractérisé en ce que

l'on condense l'acide mandélique

avec un dérivé de l'acide hydroxy phénylacétique de formule générale II,

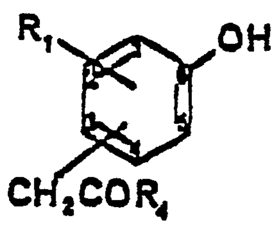

(II)

dans laquelle $R_1$ a la même signification précédemment définie pour la formule I,

$R_4$ représente un radical hydroxyle ou un radical amino de formule III

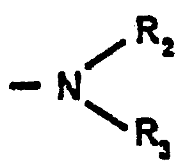

(III)

dans laquelle $R_2$ et $R_3$ ont la signification précédemment définie pour la formule I,
et soit $R_1$ est en position 1 et le radical $-CH_2COR$ est en position 3, soit $R_1$ est en position 3 et le radical $-CH_2COR_4$ est en position 1,
pour fomer,
soit les composés de formule générale I quand $R_4$ est un radical amino de formule III,
soit un acide de formule générale IV,

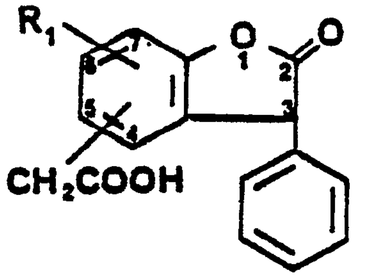

(IV)

dans laquelle la signification de $R_1$ reste identique à celle donnée précédemment et soit $R_1$ est en position 7 et le radical carboxyméthylène en position 5, soit $R_1$ est en position 5 et le radical carboxyméthylène en position 7,
que l'on soumet ensuite à l'action du chlorure de thionyle pour former un composé de formule générale V,

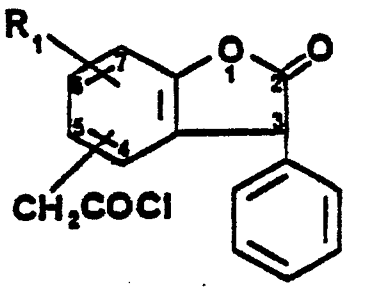

(V)

dans laquelle la signification de $R_1$ reste identique à celle donnée ci-dessus et soit $R_1$ se trouve en position 7 et le radical chloroformylméthylène en position 5, soit $R_1$ se trouve en position 5 et le radical chloroformylméthylène en position 7,
que l'on condense avec une amine de formule générale VI,

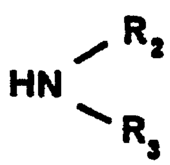

(VI)

dans laquelle $R_2$ et $R_3$ ont la signification précédemment définie pour la formule I,
pour obtenir les composés de formule générale I,
que l'on peut ensuite, si l'on désire, salifier avec un acide minéral ou organique pharmaceutiquement acceptable.

Les composés de formule générale II sont préparés lorsqu'ils sont des dérivés de l'hydroxy-2 phénylacétamide selon des procédés classiques (Bericht. (1895) 28, p. 989) et lorsqu'ils sont des dérivés de l'hydroxy-4 phénylacétamide selon les procédés décrits dans Chem.Abst. 50, 4990i ou dans J.Am.Chem.Soc. (1946) p 2633.

La condensation de l'acide mandélique avec les composés de formule générale II s'effectue en milieu acide et à une température entre 50°C et 120°C.

Les halogénures d'acyles de formule générale V sont préparés à une température entre 50°C et 100°C dans un solvant organique anhydre apolaire, en faisant réagir le chlorure de thionyle sur les acides de formule générale IV.

La condensation des amines de formule générale VI avec les composés de formule générale V s'effectue en milieu alcalin dans un solvant organique apolaire anhydre et à la température ambiante.

Parmi les acides pharmaceutiquement acceptables pour la préparation des sels d'addition aux composés de formule générale I, on peut citer les acides chlorhydrique, phosphorique, citrique, oxalique, sulfurique, tartrique, maléïque, mandélique, méthane-sulfonique, etc...

Les composés selon l'invention, ainsi que leurs sels sont doués de propriétés pharmacologiques fort intéressantes et se distinguent des autres dérivés de dihydro-2,3 oxo-2 benzofuranne déjà connus. En effet, les essais pharmacologiques in vivo ont montré que les composés de la présente invention exercent un puissant effet antihypoxique chez l'animal.

Lors du vieillissement ou à la suite d'un accident vasculaire cérébral, la fragilité et la vulnérabilité cellulaire accrues sont des composantes physiopathologiques importantes pour la recherche de nouvelles thérapeutiques visant à protéger le cerveau mis en situation d'incapacité à répondre à toute nouvelle agression issue de son environnement.

Une telle agression peut être reproduite sous forme d'un défaut d'apport en oxygène et c'est pourquoi, par leurs conséquences, une analogie étroite existe entre l'hypoxie et le vieillissement cérébral. Cette analogie s'exprime notamment par la baisse des réserves énergétiques du cerveau, la moindre résistance au stress et la baisse du renouvellement de la synthèse oxygéno-dépendante des neurotransmetteurs.

Les composés de la présente invention ont été testés sur leur capacité à prolonger la survie du tissu cérébral lors d'hypoxie aiguë chez la souris, ou à maintenir le taux de composés énergétiques tissulaires chez le rat soumis à une baisse d'apport en oxygène (Pharmacology of Cerebral Ischemia, (1968) p 334-339 Elservier Science Publishers B.V., J Krieglstein edt). Dans les deux types d'expérience réalisée, les composés de l'invention ont été comparés à des composés de référence qui sont les méclofénoxate, le pyritinol et le piracétam (Arz. Forsch. Drug Res. (1986), 36 II N°9, p. 1314-1320).

Ces derniers ont été choisis pour leurs indications thérapeutiques vis-à-vis des symptômes associés à la sénescence ou aux séquelles d'accident vasculaire cérébral, indications revendiquées sur la base de l'effet antihypoxique et nootrope sans intervention d'effet vasculaire. Les composés de type myolytique ou adrénolytique ont donc été écartés.

Les essais pharmacologiques chez la souris ont prouvé que les composés de la présente invention ont un effet protecteur antihypoxique 2 à 4 fois plus puissant par rapport à celui du composé de référence le plus actif. Chez le rat hypoxié, les composés de l'invention ont exercé les mêmes effets protecteurs sur l'énergie cérébrale que les composés de référence, mais à des doses qui sont au moins 3 fois inférieures et ont ainsi confirmé le grand intérêt de leur emploi en thérapeutique.

En s'opposant nettement à la mort cérébrale et à la défaillance énergétique tissulaire lors d'insuffisance d'apport en oxygène, les composés de la présente invention exercent un effet antihypoxique marqué et sont donc utiles dans les cas de syndromes ischémiques de toute localisation aiguë, transitoire ou progressive puisqu'ils exercent leurs propriétés pharmacologiques vis-à-vis de la défaillance d'oxygénation qui accompagne ces accidents. Leurs propriétés pharmacologiques permettent leur application dans la correction des désordres liés à l'hypoxémie et à l'insuffisance énergétique par exemple lors du vieillissement cérébral.

L'invention s'étend aussi aux compositions pharmaceutiques renfermant comme principe actif au moins un composé de formule générale I ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie d'administration.

La voie d'administration préférée est la voie orale ou parentérale. D'une manière générale, la posologie unitaire s'échelonnera entre 0,5 et 300 mg et la posologie journalière, utilisable en thérapeutique humaine, entre 0,5 et 900 mg.

Les exemples suivants, donnés à titre non-limitatif, illustrent l'invention.

Les points de fusion indiqués sont mesurés selon la technique Micro-Köfler. Les spectres infra-rouge sont obtenus avec des solutions des produits dans le Nujol. Les spectres de résonance magnétique nucléaire du proton (R.M.N.) ont été enregistrés à 60 MHz.

**EXEMPLE 1**

**(dihydro-2,3 oxo-2 phényl-3 benzofurannyl-5)-2 acétamide**

A un mélange de 16 g d'acide mandélique et 15 g de hydroxy-4 phénylacétamide (J.Am.Chem.Soc (1946) p 2633), on ajoute 190 ml d'une solution d'acide sulfurique à 80%. On chauffé le milieu à 80°C très rapidement et on hydrolyse aussitôt à l'aide de 600 g de glace. On extrait la phase aqueuse avec 3 fois 200 ml de chloroforme, on lave la phase organique avec une solution saturée de bicarbonate de sodium, on la sèche sur sulfate de sodium anhydre et on la concentre sous vide. On recristallise le produit brut obtenu dans le chloroforme.

Rendement: 20%

Point de fusion: 162°C

Les constantes physiques spectrales de ce composé sont indiquées dans le Tableau I.

**EXEMPLE 2**

**N-benzyl (dihydro-2,3 oxo-2 phényl-3 benzofurannyl-5)-2 acétamide**

STADE A

N-benzyl hydroxy-4 phénylacétamide

Un mélange de 80 g d'ester méthylique de l'acide hydroxy-4 phénylacétique et de 51,6 g de benzylamine est porté à 180°C pendant 8 heures. On laisse le milieu revenir à température ambiante, on filtre les cristaux, on les lave à l'éthanol puis à l'éther éthylique.
Rendement: 55%
Point de fusion: 149-150°C

STADE B

A un mélange de 53,4 g d'amide obtenu ci-dessus et de 26,2 g d'acide mandélique, on ajoute 200 ml d'acide sulfurique à 85%. On porte le plus rapidement possible, le milieu à 120°C en agitant. On hydrolyse à l'aide d'un mélange glace et acétate d'éthyle (50:50 V/V). On lave la phase organique à l'aide d'une solution saturée de bicarbonate de sodium puis à l'eau, on la sèche sur sulfate de magnésium anhydre et on la concentre sous vide. On obtient 36 g de produit solide brut que l'on recristallise dans un mélange éthanol-eau.
Rendement: 15%
Point de fusion: 164°C

Les constantes physiques spectrales de ce composé sont indiquées dans le Tableau I.

**EXEMPLE 3**

**(dihydro-2,3 oxo-2 phényl-3 benzofurannyl-5)-2 [(trifluorométhyl-5 pyridinyl-2)-4 pipérazinyl]-1 oxo-1 éthane**

STADE A

Acide (dihydro-2,3 oxo-2 phényl-3 benzofurannyl-5)-2 acétique.

On mélange dans un ballon 75 g d'acide mandélique et 75 g d'acide hydroxy-4 phénylacétique. On ajoute 800 ml d'acide sulfurique à 80% et on porte alors rapidement le milieu à 90°C en agitant. On hydrolyse sur 2 kg de glace, on extrait la phase aqueuse avec 3 fois 500 ml d'acétate d'éthyle, on lave la phase organique à l'eau, on la sèche sur sulfate de sodium anhydre et on la concentre sous vide. On obtient ainsi 117 g d'une huile jaune que l'on purifie par chromatographie sur 1200 g de silice de 70-230 mesh en utilisant comme éluant un mélange de dichlorométhane et de méthanol (98,5:1,5 V/V). Aprés évaporation du solvant, on obtient le produit pur.
Rendement: 50%
Point de fusion: 136°C

Spectre RMN du proton du produit en solution dans le DMSO-$d_6$:
12,5 à 13 ppm,m, 1H échangeable; 7,1 à 7,7 ppm,m,8H; 5,4 ppm,s,1H; 3,6 ppm,s,2H.

STADE B

Chlorure de (dihydro-2,3 oxo-2 phényl-3 benzofurannyl-5)-2 acétyle

A une solution de 67 g de l'acide obtenu précédemment dans 600 ml de benzène anhydre agitée et chauffée à 70°C, on ajoute goutte à goutte 36,3 ml de chlorure de thionyle bidistillé. On chauffe ensuite le milieu à reflux pendant 2 heures, on évapore le solvant sous vide et on reprend l'huile résiduelle 3 fois avec 150 ml de benzène anhydre, que l'on évapore ensuite sous vide. On lave le produit cristallisé dans l'éther de pétrole, et on le filtre.

Rendement: 95%

STADE C

On agite à 5°C une solution de 7,4 g d'halogénure d'acétyle obtenu au stade précédent dans 300 ml de benzène anhydre et on ajoute goutte à goutte un mélange de 7,85 g de triéthylamine et 4,1 g de dichlorhydrate de (trifluorométhyl-5 pyridinyl-2)-4 pipérazine. On agite à température ambiante pendant 45 minutes, on lave le mélange réactionnel 2 fois avec 100 ml d'eau, on sèche la phase organique sur sulfate de sodium anhydre, on évapore le solvant sous vide et on purifie les 10,5 g d'huile résiduelle obtenue par chromatographie sur silice de 230-400 mesh en utilisant comme éluant un mélange de dichlorométhane et de méthanol (99:1 V/V)/ Après évaporaton du solvant, on obtient des cristaux purs.
Rendement: 35%
Point de fusion: 55°C
Les constantes physiques spectrales du (dihydro-2,3 oxo-2 phényl-3 benzofurannyl-5)-2 [(trifluorométhyl-5 pyridinyl-2)-4 pipérazinyl]-1 oxo-1 éthane sont indiquées dans le Tableau I.

**EXEMPLE 4**

**(dihydro-2,3 oxo-2 phényl-3 benzofurannyl-5)-2 [(méthylènedioxy-3,4 benzyl)-4 pipérazinyl]-1 oxo-1 éthane**

Ce composé a été préparé selon le procédé décrit dans l'exemple 3, en utilisant au Stade C la (méthylènedioxo-3,4 benzyl)-4 pipérazine.
Rendement: 48%
Point de fusion: environ 70°C (meringue).
Les constantes physiques spectrales de ce composé sont indiquées dans le Tableau I.

**EXEMPLE 5**

**Tartrate du (dihydro-2,3 oxo-2 phényl-3 benzofurannyl-5)-2 (méthyl-4 pipérazinyl)-1 oxo-1 éthane**

Le (dihydro-2,3 oxo-2 phényl-3 benzofurannyl-5)-2 (méthyl-4 pipérazinyl)-1 oxo-1 éthane a été préparé selon le procédé décrit dans l'exemple 3 mais en utilisant au Stade C la méthyl-4 pipérazine. Ce dernier composé a été dissout dans une solution d'acide d,l tartrique pour former le sel correspondant, et ensuite recristallisé dans l'éther éthylique.
Rendement: 38%
Point de fusion du sel: 108°C
Les constantes physiques spectrales de ce sel sont indiquées dans le Tableau I.

**TABLEAU I**

**COMPOSES DE FORMULE**

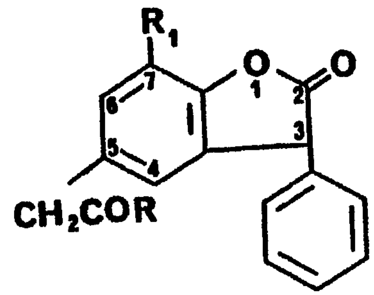

| Ex | $R_1$ | R | IR ($cm^{-1}$) |  | RMN (solvant) |
|---|---|---|---|---|---|
|  |  |  | ν C = 0 lactone | ν C = 0 extracyclique |  |
| 1 | -H | $-NH_2$ | 1800 | 1655 | ($CDCl_3$ + DMSO-$d_6$)<br>7,1 à 7,6 ppm,m,8H; 6,2 à 6,8 ppm,m,2H échangeables; 5,5 ppm,s,1H; 3,4 ppm,s,2H |
| 2 | -H | 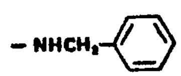 | 1810 | 1640 | ($CDCl_3$ + DMSO-$d_6$)<br>7 à 7,4 ppm,m,13H + 1H échangeable;<br>4,8 ppm,s,1H; 4,3 ppm,d,2H;<br>3,4 ppm,S,2H |

**TABLEAU I**
**(SUITE 1)**

| Ex | $R_1$ | R | IR ($cm^{-1}$) $\nu C=0$ lactone | IR ($cm^{-1}$) $\nu C=0$ extracyclique | RMN (solvant) |
|---|---|---|---|---|---|
| 3 | - H | 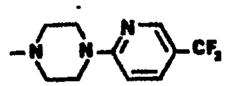 | 1810 | 1645 | ($CDCl_3$)<br>8,4 à 8,7 ppm,m,1H; 7,6 ppm,d,1H; 7 à 7,5 ppm,m,8H; 6,6 ppm,d,1H; 4,9 ppm,s,1H; 3,7 ppm,s,2H; 3,4 à 3,6 ppm,m,8H |
| 4 | - H | 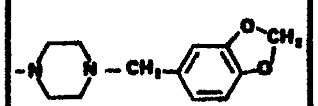 | 1810 | 1640 | ($CDCl_3$)<br>6,8 à 7,8 ppm,m,11H; 6,05 ppm,s,2H; 5 ppm,s,1H; 3,7 ppm,s,2H; 3,3 à 3,6 ppm,m,4H; 3,4 ppm,s,2H; 2,1 à 2,5 ppm,m,4H |
| 5 | - H | 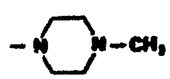 | 1805 | 1725 (acide)<br>1650 (amide) | ($D_2O$)<br>7 à 8 ppm,m,8H; 5,5 ppm,s,1H; 4,7 ppm,s,2H; 4,6 ppm,s,2H; 2,8 à 4,6 ppm,m,11H |

EXEMPLE 6

<u>(dihydro-2,3 oxo-2 phényl-3 benzofurannyl-7)-2 morpholino-1 oxo-1 éthane</u>

STADE A

<u>(hydroxy-2 phényl)-2 morpholino-1 oxo-1 éthane</u>

A une solution de 30 g de dihydro-2,3 benzofurannone-2 dans 100 ml d'éthanol, on ajoute en une seule fois 27,3 g de morpholine et on agite le milieu pendant 1 heure. On évapore le solvant sous vide, on reprend la gomme résiduelle dans l'éther isopropylique à chaud et on laisse le milieu revenir à température ambiante. On filtre le produit cristallisé, on le lave à l'éther isopropylique, on le sèche. On obtient 45 g de produit cristallisé blanc.
Point de fusion: 122°C

STADE B

On mélange dans un erlenmeyer 30,5 g du produit obtenu au stade précédent et 23 g d'acide mandélique. On ajoute à température ambiante 150 ml d'acide sulfurique à 85% et on porte le milieu à 120°C le plus rapidement possible en agitant. On plonge immédiatement le mélange réactionnel dans 1 litre d'un mélange acétate d'éthyle et glace (50:50 V/V). On décante, on rassemble les phases organiques, on les lave avec une solution saturée de bicarbonate de sodium, on sèche la phase organique sur sulfate de sodium anhydre, on évapore le solvant sous vide et on recristallise le produit brut dans un mélange d'éthanol et d'eau (60:40 V/V) pour recueillir des cristaux blancs.
Rendement: 22%
Point de fusion: 147°C
Les constantes physiques spectrales de ce composé sont indiquées dans le Tableau II.

**EXEMPLES 7 et 8**

Ces composés ont été préparés selon le procédé décrit dans l'exemple 6 mais en condensant au Stade A la benzofurannone-2 avec les amines adéquates. Les constantes physiques spectrales de ces amides sont indiquées dans le Tableau II.

**EXEMPLE 7**

**<u>N,N-diméthyl (dihydro-2,3 oxo-2 phényl-3 benzofurannyl-7)-2 acétamide</u>** Rendement: 18%
Point de fusion: 134°C

**EXEMPLE 8**

**<u>(dihydro-2,3 oxo-2 phényl-3 benzofurannyl-7)-2 acétamide</u>** Rendement: 30%
Point de fusion: 218°C

**EXEMPLE 9**

**<u>(chloro-5 dihydro-2,3 oxo-2 phényl-3 benzofurannyl-7)-2 acétamide</u>**

Ce composé a été préparé selon le procédé décrit dans l'exemple 6 mais en utilisant au Stade A la chloro-5 dihydro-2,3 benzofurannone-2 et l'ammoniaque.
Rendement: 45%
Point de fusion: 162°C
Ces constantes physiques spectrales sont indiquées dans le Tableau II.

**TABLEAU II**

**COMPOSES DE FORMULE**

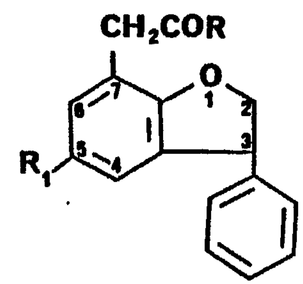

| Ex | $R_1$ | R | IR ($cm^{-1}$) $\nu C=0$ lactone | IR ($cm^{-1}$) $\nu C=0$ extracyclique | RMN (solvant) |
|---|---|---|---|---|---|
| 6 | -H | 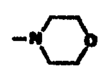 | 1805 | 1650 | ($CDCl_3$) 7,1 à 7,5 ppm,m,8H; 4,9 ppm,s,1H; 3,75 ppm,s,2H; 3,65 ppm,s,8H |
| 7 | -H | 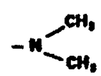 | 1810 | 1640 | ($CDCl_3$) 7 à 7,4 ppm,m,8H; 4,9 ppm,s,1H; 3,75 ppm,s,2H; 3,05 ppm,s,3H; 2,95 ppm,s,3H |
| 8 | -H | $-NH_2$ | 1800 | 1650 | ($CDCl_3$ + DMSO-$d_6$) 7 à 7,5 ppm,m,8H; 6 à 6,9 ppm,m,2H échangeables; 5,3 ppm,s,1H; 3,6 ppm,s,2H |
| 9 | -Cl | $-NH_2$ | 1810 | 1655 | ($CDCl_3$ + DMSO-$d_6$) 7,1 à 7,6 ppm,m,7H; 5,5 à 6,5 ppm,m,2H échangeables; 5 ppm,s,1H; 3,55 ppm,s,2H |

## ETUDE PHARMACOLOGIQUE

### EXEMPLE 10

**Hypoxie aiguë chez la souris**

Des souris CD1 (Charles River), de sexe mâle, ayant reçu 30 minutes auparavant par voie intropéritonéale le composé à tester ou un composé de référence, sont soumises à une hypoxie aiguë de type hypobare. Pour cela, elles sont placées dans une enceinte où la pression atmosphérique peut être abaissée rapidement (en 30 secondes) à une valeur de 160 mbar, ce qui provoque la mort de tous les animaux, 15 secondes environ après l'obtention de cette pression hypoxique.

La survie du cerveau est appréciée par la mesure du temps d'apparition du dernier gasp respiratoire.

Le temps de survie d'un lot traité est comparé à celui d'un lot témoin ne recevant que le solvant.

Le pourcentage d'augmentation du temps de survie après traitement des animaux avec les composés de l'invention est indiqué dans le tableau III. Les résultats significatifs sont soulignés ($p < 0.05$).

Comme le tableau III le démontre, les composés de l'invention exercent un puissant effet anti-hypoxique très supérieur à celui des c omposés de référence. En effet, à la dose de 100 mg/kg, le méclofenoxate, le pyritinol et le piracétam n'augmentent le temps de survie cérébrale des animaux que de 22%, 27% et 11% respectivement. La protection n'est significative que pour le pyritinol.

A la même dose, les composés de l'invention ont un effet protecteur, beaucoup plus puissant que ce dernier composé. L'augmentation du temps de survie est par exemple de 170% pour le composé de l'exemple 1, 133% pour le composé de l'exemple 4, 111% pour le composé de l'exemple 5.

**TABLEAU III**

**Pourcentage d'augmentation du temps de survie**

| COMPOSES | DOSES mg/kg (I.P.) | | | |
|---|---|---|---|---|
| | 3 | 10 | 30 | 100 |
| Meclofenoxate | | | + 4 | + 22 |
| Pyritinol | | | + 13 | + 27 |
| Piracetam | | | | + 11 |
| Ex. 1 | + 24 | | + 46 | + 170 |
| Ex. 3 | | | + 36 | + 57 |
| Ex. 4 | + 43 | + 49 | + 54 | + 133 |
| Ex. 5 | | | | + 111 |
| Ex. 8 | | | | + 13 |
| Ex. 9 | | | | + 23 |

### EXEMPLE 11

**Hypoxie aiguë chez le rat**

Des rats Fischer 344 (Charles River), de sexe mâle, ayant reçu 30 minutes auparavant le composé à tester ou un composé de référence, sont soumis à un défaut d'apport en oxygène en étant placés dans

une enceinte normobare dans laquelle la composition du mélange gazeux circulant peut être changée avec précision. Alors que les rats témoins respirent un mélange gazeux contenant 21% d'oxygène et 79% d'azote, les rats hypoxiés respirent durant 2 minutes un mélange de 3% d'oxygène et 97% d'azote.

A la fin de la période hypoxique, les animaux sont rapidement sacrifiés par immersion totale dans l'azote liquide. Le cerveau congelé est prélevé et les composés énergétiques (ATP, ADP, AMP) sont extraits et dosés par la méthode de luminescence de la luciférine.

La charge énergétique tissulaire (EC) est calculée selon la formule d'ATKINSON:

$$EC = \frac{ATP + \frac{1}{2} ADP}{ATP + ADP + AMP}$$

Les résultats de cette étude sont indiqués dans le tableau IV.

L'hypoxie entraîne chez les animaux témoins, un effondrement du taux d'ATP tissulaire (-74,2%) qui s'accompagne d'une hausse des composés mono- et di-phosphatés (AMP, ADP). C'est ce que traduit la baise de la charge énergétique totale (-25,3%).

A la dose de 300 mg/kg, le piracétam n'exerce que très peu de protection en inhibant de 3,9% seulement les effets de l'hypoxie sur le taux d'ATP.

A la même dose, le méclofenoxate inhibe de 59,7% et de 69,6% les effets de l'hypoxie respectivement sur le taux d'ATP et la charge énergétique. Un tel effet est observé avec une dose de 30 mg/kg du composé de l'exemple 1.

Dans les mêmes conditions, le pyritinol à la dose de 100 mg/kg n'exerce qu'un effet plus modeste. La chute d'ATP est inhibée de 23% tandis que celle de la charge énergétique est inhibée de 35%.

**TABLEAU IV**

**HYPOXIE AIGUE CHEZ LE RAT**

| | | | |
|---|---|---|---|
| Rats témoins en normoxie | : | ATP = 2,373 µmoles/g | EC = 0,959 |
| Rats témoins en hypoxie | : | ATP = - 74,2 % | EC = - 25,3% |

| COMPOSES | DOSE mg/kg I.P. | % D'INHIBITION DES EFFETS DE L'HYPOXIE | |
|---|---|---|---|
| | | ATP | EC |
| Méclofenoxate | 300 | 59,7 | 69,6 |
| Pyritinol | 030<br>100 | 11,1<br>23,3 | 16,6<br>35,2 |
| Piracétam | 300 | 3,9 | 1,6 |
| Exemple 1 | 030 | 64,6 | 66 |

PREPARATION PHARMACEUTIQUE

| Exemple 12 | |
|---|---|
| Gélules dosées à 20 mg de (dihydro-2,3 oxo-2 phényl-3 benzofurannyl-5)-2 acétamide | |
| (dihydro-2,3 oxo-2 phényl-3 benzofurannyl-5-)-2 acétamide | 20 mg |
| Amidon de maïs | 15 mg |
| Lactose | 25 mg |
| Talc | 5 mg |
| Pour une gélule N° 3. | |

## Revendications

**1.** Composés de formule générale I,

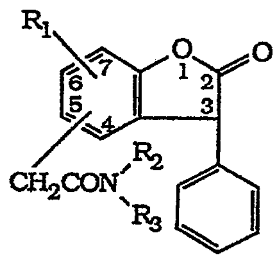

(I)

dans laquelle:
ou $R_1$ est en position 7, et le groupement acétamido est en position 5, ou $R_1$ est en position 5, le groupement acétamido est en position 7,
$R_1$ représente un atome d'hydrogène ou d'halogène,
$R_2$ et $R_3$ identiques ou différents représentent chacun un atome d'hydrogène ou un radical alkyle linéaire ou ramifié contenant 1 à 4 atomes de carbone, un radical benzyle éventuellement substitué par un atome d'halogène par un radical alcoxy contenant 1 à 4 atomes de carbone ou par un radical alkyle de 1 à 4 atomes de carbone, un radical méthylènedioxy-3,4 benzyle, ou forment ensemble avec l'azote auquel ils sont attachés un radical morpholinyle-4, ou un radical pipérazinyle-1 (éventuellement substitué en 4 par un radical alkyle de 1 à 4 atomes de carbone, un radical benzyle éventuellement substitué par un radical alkyle de 1 à 4 atomes de carbone, un radical méthylènedioxy-3,4 benzyle, ou un radical pyridyle-2 éventuellement substitué par un radical alkyle de 1 à 4 atomes de carbone ou un radical trifluorométhyle), et leurs sels d'addition à un acide minéral ou organique, pharmaceutiquement acceptable.

**2.** Composés de formule générale I′,

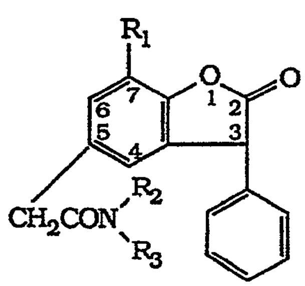

(I′)

dans laquelle
$R_1$ représente un atome d'hydrogène ou d'halogène,
$R_2$ et $R_3$ identiques ou différents représentent chacun un atome d'hydrogène ou un radical alkyle linéaire ou ramifié contenant 1 à 4 atomes de carbone, un radical benzyl éventuellement substitué par un atome d'halogène par un radical alcoxy contenant 1 à 4 atomes de carbone ou par un radical alkyle de 1 à 4 atomes de carbone, un radical méthylènedioxy-3,4 benzyle, ou forment ensemble avec l'azote auquel ils sont attachés un radical morpholinyle-4, ou un radical pipérazinyle-1 (éventuellement substitué en 4 par un radical alkyle de 1 à 4 atomes de carbone, un radical benzyle éventuellement substitué par un radical alkyle de 1 à 4 atomes de carbone, un radical méthylènedioxy-3,4 benzyle, ou un radical pyridyle-2 éventuellement substitué par un radical alkyle de 1 à 4 atomes de carbone ou un radical trifluorométhyle),

et leurs sels d'addition à un acide minéral ou organique, pharmaceutiquement acceptable.

**3.** Le (dihydro-2,3 oxo-2 phényl-3 benzofurannyl-5)-2 acétamide et ses sels d'addition avec un acide minéral ou organique, pharmaceutiquement acceptable.

**4.** Procédé de préparation des composés de formule générale I selon la revendication 1, caractérisé en ce que l'on condense l'acide mandélique
avec un dérivé de l'acide hydroxy phénylacétique de formule générale II,

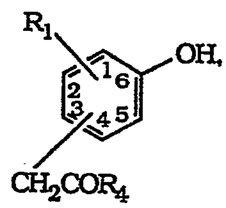

(II)

dans laquelle $R_1$ a la même signification précédemment définie pour la formule I, $R_4$ représente un radical hydroxyle ou un radical amino de formule III

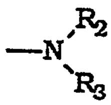

(III)

dans laquelle $R_2$ et $R_3$ ont la signification précédemment définie pour la formule I,
et soit $R_1$ est en position 1 et le radical $-CH_2COR_4$ est en position 3, soit $R_1$ est en position 3 et le radical $-CH_2COR_4$ est en position 1,
pour former,
soit les composés de formule générale I quand $R_4$ est un radical amino de formule III,
soit un acide de formule générale IV,

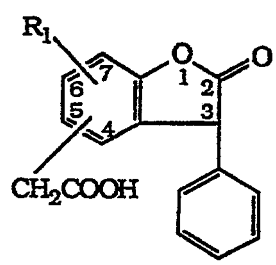

(IV)

dans laquelle la signification de $R_1$ reste identique à celle donnée précédemment et soit $R_1$ est en position 7 et le radical carboxyméthylène en position 5, soit $R_1$ est en position 5 et le radical carboxyméthylène en position 7,
que l'on soumet ensuite à l'action du chlorure de thionyle pour former un composé de formule générale V,

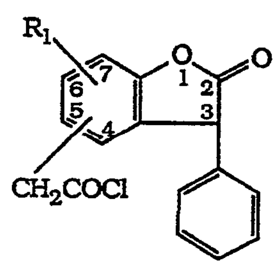

(V)

dans laquelle la signification de $R_1$ reste identique à celle donnée ci-dessus et soit $R_1$ se trouve en position 7 et le radical chloroformylméthylène en position 5, soit $R_1$ se trouve en position 5 et le radical chloroformylméthylène en position 7,
que l'on condense avec une amine de formule générale VI,

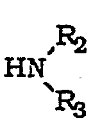

(VI)

dans laquelle $R_2$ et $R_3$ ont la signification précédemment définie pour la formule I,
pour obtenir les composés de formule générale I,
que l'on peut ensuite, si l'on désire, salifier avec un acide minéral ou organique pharmaceutiquement acceptable.

**5.** Composition pharmaceutique contenant comme principe actif un composé selon l'une quelconque des revendications 1 à 3, en association ou en mélange avec un excipient ou un véhicule inerte non toxique, pharmaceutiquement acceptable.

**6.** Composition pharmaceutique selon la revendication 5 renfermant comme principe actif au moins un composé selon les revendications 1 à 3 utilisable dans le traitement des maladies liées à l'hypoxémie et à l'insuffisance énergétique ou le vieillissement cérébral.

**Patentansprüche**

1. Verbindung der allgemeinen Formel

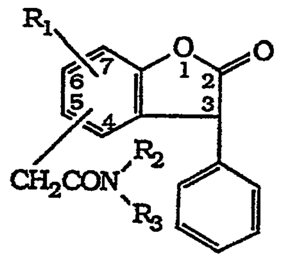

(I)

worin
entweder $R_1$ in der Stellung 7 und die Acetamido-gruppe in der Stellung 5 oder die Gruppe $R_1$ in der Stellung 5 und die Acetamido-gruppe in der Stellung 7 stehen,
$R_1$ ein Wasserstoffatom oder ein Halogenatom und
$R_2$ und $R_3$, die gleichartig oder verschieden sein können, jeweils ein Wasserstoffatom oder eine geradkettige oder verzweigte Alkylgruppe mit 1 bis 4 Kohlenstoffatomen, eine gegebenenfalls durch ein Halogenatom, eine Alkoxygruppe mit 1 bis 4 Kohlenstoffatomen oder eine Alkylgruppe mit 1 bis 4 Kohlenstoffatomen substituierte Benzylgruppe, eine 3,4-Methylendioxy-benzylgruppe oder gemeinsam mit dem Stickstoffatom, an das sie gebunden sind, eine Morpholin-4-yl-gruppe oder eine Piperazin-1-yl-gruppe (die gegebenenfalls in der 4-Stellung durch eine Alkylgruppe mit 1 bis 4 Kohlenstoffatomen substituierte Benzylgruppe, eine gegebenenfalls durch eine Alkylgruppe mit 1 bis 4 Kohlenstoffatomen, eine 3,4-Methylendioxy-benzolgruppe oder eine Pirid-2-yl-gruppe, die gegebenenfalls durch eine Alkylgruppe mit 1 bis 4 Kohlenstoffatomen oder eine Trifuormethylgruppe substituiert sein kann, substituiert ist), und deren Additionssalze mit einer pharmazeutisch annehmbaren anorganischen oder organischen Säure.

2. Verbindungen der allgemeinen Formel I'

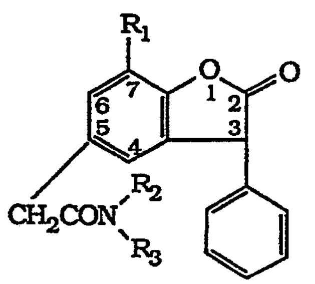

(I')

in der
$R_1$ ein Wasserstoffatom oder ein Halogenatom und
$R_2$ und $R_3$, die gleichartig oder verschieden sein können, jeweils ein Wasserstoffatom oder eine geradkettige oder verzweigte Alkylgruppe mit 1 bis 4 Kohlenstoffatomen, eine gegebenenfalls durch ein Halogenatom, eine Alkoxygruppe mit 1 bis 4 Kohlenstoffatomen oder eine Alkylgruppe mit 1 bis 4 Kohlenstoffatomen substituierte Benzylgruppe, eine 3,4-Methylendioxy-benzyl-gruppe oder gemeinsam mit dem Stickstoffatom, an das sie gebunden sind, eine Morpholin-4-yl-gruppe oder eine Piperazin-1-yl-gruppe (die gegebenenfalls in der 4-Stellung durch eine Alkylgruppe mit 1 bis 4 Kohlenstoffatomen substituierte Benzylgruppe, eine 3,4-Methylendioxy-benzolgruppe oder eine Pirid-2-yl-gruppe, die gegebenenfalls durch eine Alkylgruppe mit 1 bis 4 Kohlenstoffatomen oder eine Trifuormethylgruppe substituiert sein kann, substituiert ist), und deren Additionssalze mit einer pharmazeutisch annehmbaren anorganischen oder organischen Säure.

3. 2-(2,3-Dihydro-2-oxo-3-phenyl-benzofuran-5-yl)-acetamid und dessen Additionssalze mit einer pharmazeutisch annehmbaren anorganischen oder organischen Säure.

4. Verfahren zur Herstellung der Verbindungen der allgemeinen Formel I gemäß Anspruch 1, dadurch gekennzeichnet, daß man Mandelsäure mit einem Derivat der Hydroxy-phenylessigsäure der allgemeinen Formel II

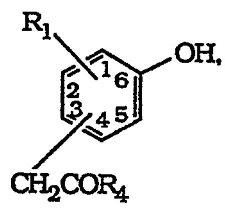
(II)

in der $R_1$ die oben bezüglich der Formel I angegebenen Bedeutung besitzt und $R_4$ eine Hydroxylgruppe oder eine Aminogruppe der Formel III

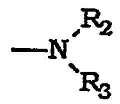
(III)

worin $R_2$ und $R_3$ die oben bezüglich der Formel I angegebene Bedeutung besitzen, bedeutet und entweder die Gruppe $R_1$ in der 1-Stellung und die Gruppe $-CH_2COR_4$ in der 3-Stellung oder die Gruppe in der 3-Stellung und die Gruppe $-CH_2COR_4$ in der 1-Stellung stehen, kondensiert zur Bildung entweder der Verbindung der allgemeinen Formel I, in der $R_4$ eine Aminogruppe der Formel III darstellt, oder einer Säure der allgemeinen Formel IV

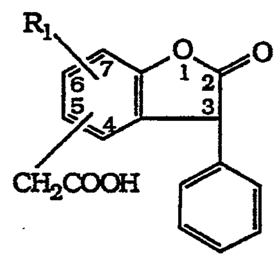
(IV)

in der $R_1$ die oben angegebenen Bedeutungen besitzt und bei der entweder die Gruppe $R_1$ in der 7-Stellung und die Carboxymethylen-gruppe in der 5-Stellung oder die Gruppe $R_1$ in der 5-Stellung und Carboxymethlen-gruppe in der 7-Stellung stehen, welche man der Einwirkung con Thionylchlorid unterwirft zur Bildung einer Verbindung der allgemeinen Formel V

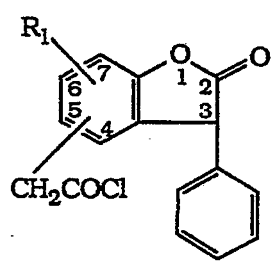
(V)

in der $R_1$ die oben angegebenen Bedeutungen besitzt und die Gruppe $R_1$ in der 7-Stellung und die Chlorformylmethylen-gruppe in der 5-Stellung oder die Gruppe $R_1$ in der 5-Stellung und die Chlorformylmethlen-gruppe in der 7-Stellung stehen, welche man mit einem Amin der allgemeinen Formel VI

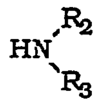
(VI)

worin $R_2$ und $R_3$ die oben bezüglich der Formel I angegebene Bedeutung besitzen, kondensiert zur Bildung der Verwendung der allgemeinen Formel I, welche man anschliessend gewünschtenfalls mit einer pharmazeutisch annehmbaren anorganischen oder organischen Säure in die Salze umwandeln kann.

5. Pharmazeutische Zubereitung als Wirkstoff eine Verbindung nach einem der Ansprüche 1 bis 3 in Kombination oder in Mischung mit einem inerten, nicht-toxischen, pharmazeutisch annehmbaren Träger und/oder Hilfsstoff.

6. Pharmazeutische Zubereitung nach Anspruch 5 enthaltend als Wirkstoff mindestens eine Verbindung nach den Ansprüchen 1 bis 3 in einer zur Behandlung von mit Hypoxämie und Energiemangelerscheinungen oder Gehirnalterung verknüpften Erkrankungen.

## Claims

1. Compounds of general formula I

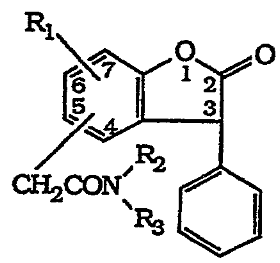

(I)

in which:

either $R_1$ is at the 7-position and the acetamido group is at the 5-position, or $R_1$ is at the 5-position and the acetamido group is at the 7-position,

$R_1$ denotes a hydrogen or halogen atom,

$R_2$ and $R_3$, which may be identical or different, each denote a hydrogen atom or a linear or branched alkyl radical containing 1 to 4 carbon atoms, a benzyl radical optionally substituted with a halogen atom, with an alkoxy radical containing 1 to 4 carbon atoms or with an alkyl radical having 1 to 4 carbon atoms, or a 3,4-methylenedioxybenzyl radical, or form, together with the nitrogen to which they are attached, a 4-morpholinyl radical or a 1-piperazinyl radical (optionally substituted at the 4-position with an alkyl radical having 1 to 4 carbon atoms, a benzyl radical optionally substituted with an alkyl radical having 1 to 4 carbon atoms, a 3,4-methylenedioxybenzyl radical, or a 2-pyridyl radical optionally substituted with an alkyl radical having 1 to 4 carbon atoms or a trifluoromethyl radical), and their addition salts with a pharmaceutically acceptable inorganic or organic acid.

2. Compounds of general formula I'

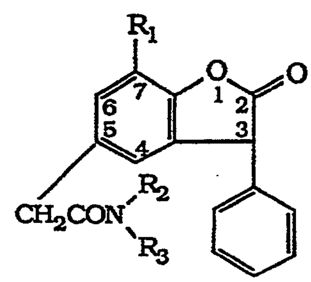

(I')

in which

$R_1$ denotes a hydrogen or halogen atom,

$R_2$ and $R_3$, which may be identical or different, each denote a hydrogen atom or a linear or branched alkyl radical containing 1 to 4 carbon atoms, a benzyl radical optionally substituted with a halogen atom, with an alkoxy radical containing 1 to 4 carbon atoms or with an alkyl radical having 1 to 4 carbon atoms, or a 3,4-methylenedioxybenzyl radical, or form, together with the nitrogen to which they are attached, a 4-morpholinyl radical or a 1-piperazinyl radical (optionally substituted at the 4-position with an alkyl radical having 1 to 4 carbon atoms, a benzyl radical optionally substituted with an alkyl radical having 1 to 4 carbon atoms, a 3,4-methylenedioxybenzyl radical, or a 2-pyridyl radical optionally substituted with an alkyl radical having 1 to 4 carbon atoms or a trifluoromethyl radical), and their addition salts with a pharmaceutically acceptable inorganic or organic acid.

3. 2-(2,3-Dihydro-2-oxo-3-phenyl-5-benzofuranyl)acetamide and its addition salts with a pharmaceutically acceptable inorganic or organic acid.

4. Process for preparing the compounds of general formula I, according to claim 1, characterized in

that mandelic acid is condensed with a hydroxyphenylacetic acid derivative of general formula II

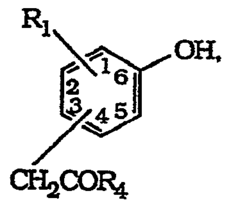

(II)

in which $R_1$ has the same meaning defined above for the formula I,

$R_4$ denotes a hydroxyl radical or an amino radical of formula III

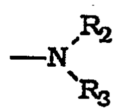

(III)

in which $R_2$ and $R_3$ have the meaning defined above for the formula I, and either $R_1$ is at the 1-position and the radical $-CH_2COR_4$ is at the 3-position, or $R_1$ is at the 3-position and the radical $-CH_2COR_4$ is at the 1 position, to form either the compounds of general formula I when $R_4$ is an amino radical of formula III, or an acid of general formula IV

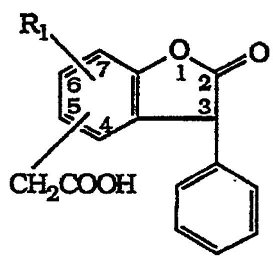

(IV)

in which the meaning of $R_1$ remains identical to that given above, and either $R_1$ is at the 7-position and the carboxymethylene radical at the 5-position, or $R_1$ is at the 5-position and the carboxymethylene radical at the 7-position, which is then subjected to the action of thionyl chloride to form a compound of general formula V

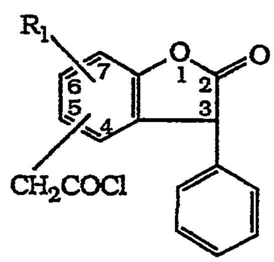

(V)

in which the meaning of $R_1$ remains identical to that given above, and either $R_1$ is at the 7-position and the chloroformylmethylene radical at the 5-position, or $R_1$ is at the 5-position and the chloroformylmethylene radical at the 7-position,

which is condensed with an amine of general formula VI

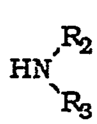

(VI)

in which $R_2$ and $R_3$ have the meaning defined above for the formula I, to obtain the compounds of general formula I, which can then, if desired, be salified with a pharmaceutically acceptable inorganic or organic acid.

5. Pharmaceutical composition containing as active principle a compound according to any one of

claims 1 to 3, in combination or as a mixture with a pharmaceutically acceptable, non-toxic, inert vehicle or excipient.

6. Pharmaceutical composition according to claim 5, containing as active principle at least one compound according to claims 1 to 3, which is usable in the treatment of diseases linked to hypoxemia and to energy insufficiency or cerebral aging.